# EUROPÄISCHE PATENTSCHRIFT

(11) **EP 1 119 641 B1**
(45) Veröffentlichungstag und Bekanntmachung des Hinweises auf die Patenterteilung: **06.12.2006**
(21) Anmeldenummer: 99952500.9
(22) Anmeldetag: 05.10.1999
(51) Int. Cl.: C12Q 1/42

(54) **VERFAHREN ZUR BESTIMMUNG VON ALKALISCHER PHOSPHATASE UNTER BESEITIGUNG VON HÄMOGLOBIN-STÖRUNGEN**
METHOD FOR DETERMINING ALKALINE PHOSPHATASE AND ELIMINATING HAEMOGLOBIN DISTURBANCES
PROCEDE POUR DETECTER LA PHOSPHATASE ALCALINE, VISANT A ELIMINER LES TROUBLES INDUITS PAR HEMOGLOBINE

(30) Priorität: 08.10.1998 DE 19846301
(43) Veröffentlichungstag der Anmeldung: 01.08.2001
(73) Patentinhaber: Roche Diagnostics GmbH, 68305 Mannheim (DE)
(72) Erfinder: WEISHEIT, Ralph, D-82380 Peissenberg (DE); TREIBER, Wolfgang, D-82362 Weilheim (DE)
(86) Internationale Anmeldenummer: PCT/EP1999/007394
(87) Internationale Veröffentlichungsnummer: WO 2000/022162

(56) Entgegenhaltungen:
- WO-A-97/45728
- DE-A- 19 622 090
- DE-A- 19 628 484
- US-A- 5 766 872
- B. HAHN ET AL.: "Polychromatic analysis: New applications of an old technique." CLINICAL CHEMISTRY., Bd. 25, Nr. 6, Juni 1979 (1979-06), Seiten 951-959, XP002129120 AMERICAN ASSOCIATION FOR CLINICAL CHEMISTRY. WINSTON., US ISSN: 0009-9147 in der Anmeldung erwähnt
- D. W. JAY ET AL.: "Characterization and mathematical correction of hemolysis interference in selected Hitachi 717 assays." CLINICAL CHEMISTRY., Bd. 39, Nr. 9, September 1993 (1993-09), Seiten 1804-1810, XP002129121 AMERICAN ASSOCIATION FOR CLINICAL CHEMISTRY. WINSTON., US ISSN: 0009-9147 in der Anmeldung erwähnt

## Beschreibung

Die Erfindung betrifft ein Verfahren zur Bestimmung von alkalischer Phosphatase in einer Probe durch optische Messung, bei dem Störungen durch freies Hämoglobin oder Blutersatzmittel mittels bestimmter Wellenlängenkombinationen beseitigt werden, eine Methode zur Beseitigung von Störungen, die durch freies Hämoglobin oder Blutersatzmittel bei der Bestimmung von alkalischer Phosphatase hervorgerufen werden, sowie die Verwendung bestimmter Wellenlängenkombinationen zur Beseitigung von Störungen durch freies Hämoglobin oder Blutersatzmittel.

Es ist bekannt, daß durch Hämolyse diagnostische Verfahren zur Bestimmung von Analyten teilweise erheblich gestört werden. Unter Hämolyse wird jegliche Zerstörung von Erythrocyten beispielsweise durch mechanische, osmotische, chemische oder enzymatische Einwirkung auf die Zellmembran der Erythrocyten verstanden. Infolge der Hämolyse tritt der Blutfarbstoff Hämoglobin (Hb) aus und ist aus einer Probe nicht mehr zu entfernen.
Problematisch ist die Anwesenheit von Hämoglobin zum einen aufgrund des mit den Spektren der nachzuweisenden Substanzen und Indikatoren (Chromogenen) zum Teil erheblich überlappenden Absorptionsspektrums von Hämoglobin, wodurch es zu Fehlmessungen in photometrischen Tests kommen kann. Zum anderen kann Hämoglobin auch mit Probenbestandteilen chemisch reagieren, wodurch ebenfalls Substanzen entstehen, die Fehlmessungen erzeugen können.

In letzter Zeit sind für Therapiezwecke beispielsweise nach hohem Blutverlust immer häufiger Blutersatzmittel im Einsatz, die auf Basis von Hämoglobin hergestellt werden. Das Hämoglobin in Blutersatzmitteln kann nativer, oder synthetischer Natur sein. Oftmals werden auch Hb-analoge Verbindungen eingesetzt. Im Gegensatz zur Hämolyse ist bei einer Therapie mit Blutersatzmitteln mit einem Hb-Gehalt im Blutserum bzw. -plasma von mehr als 2000 mg/dl zu rechnen. Störungen in Proben, die Blutersatzmittel enthalten, sind daher oftmals wesentlich ausgeprägter als in hämolytischen Proben. da das Hämoglobin oder das synthetische Analogon von vornherein in freier Form vorliegt.

Besonders gravierend ist der Störeinfluß von freiem Hämoglobin bei der photometrischen Bestimmung von alkalischer Phosphatase. Bei der Bestimmung der alkalischen Phosphatase wird die Bildung von 4-Nitrophenol bei 415 nm gemessen (Extinktionszunahme). Hämoglobin absorbiert ebenfalls bei 415 nm. In Gegenwart von Hämoglobin wird die Bestimmung der alkalischen Phosphatase in zweierlei Hinsicht gestört: Zum einen ändert sich im alkalischen Milieu das Hb-Spektrum zeitabhängig (Extinktionsabnahme), zum anderen wird ab einem bestimmten Hb-Gehalt die Photometergrenze des Meßgerätes erreicht.

Zur Beseitigung spektraler und chemischer Einflüsse von Hämoglobin auf die Analyse von Serum- oder Plasmaproben sind im Stand der Technik unterschiedliche Verfahren publiziert.

Wegen der einfachen Handhabung bei automatisierten Analysengeräten wird neben der ersten Meßwellenlänge (Hauptwellenlänge) häufig eine zweite Meßwellenlänge (Nebenwellenlänge) benutzt, mittels derer der Störeinfluß interferierender Substanzen wie Hämoglobin, Bilirubin und Lipämie beseitigt oder zumindest minimiert werden kann. In Clin Chem 25/6, 951-959 (1979) verweisen Hahn et al. darauf, daß die Nebenwellenlänge so zu wählen ist, daß diese nahe des Absorptionsminimums des Chromogens und nahe des Absorptionsmaximums der Störsubstanz liegen sollte. Die Bestimmung von alkalischer Phosphatase kann jedoch mit den hier angegebenen Meßverfahren nicht entstört werden.

Jay und Provasek beschreiben in Clin Chem 39/9, 1804-1810 (1993), daß die Hämoglobin-störung bei der Bestimmung von alkalischer Phosphatase durch eine zeitabhängige Änderung des Hb-Spektrums verursacht wird. Diese Störung kann durch mathematische Korrekturalgorithmen (Bestimmung der Hb-Konzentration in der Probe und Korrektur des gemessenen Wertes für alkalische Phosphatase um einen bestimmten Betrag, der der gemessenen Hb-Menge äquivalent ist) beseitigt werden.

Die von Jay und Provasek erwähnte mathematische Korrektur beseitigt zwar den Hb-Einfluß bis mindestens 800 mg/dl Hb, ist jedoch wenig anwenderfreundlich, da sie eine zusätzliche Messung des Hb-Gehaltes voraussetzt und anschließend noch einen mathematischen Korrekrurschritt erfordert.

Jay und Provasek (supra) beschreiben eine weitere Methode zur Entstörung durch sogenannte Rate-Blank-Messung. Die Korrektur von Hämolyse-Störungen durch Rate-Blank-Messungen ist auch in der EP-A-0 695 805 beschrieben. Hier wird vor der eigentlichen photometrischen Bestimmung einer in der Probe enthaltenen Komponente die Probe einer Vorreaktion unterworfen, durch die der Hämolysegrad der Probe bestimmt wird. Der nachfolgend erhaltene Meßwert wird dann korrigiert um einen Wert, der durch Korrelation des Hämolysegrades mit dem Meßfehlerbeitrag von störenden Komponenten ermittelt wurde.

Durch Rate-Blank-Messungen kann die Hb-Störung beseitigt werden, allerdings nur bis zu einem Hb-Gehalt von ca. 1200 mg/dl, da bei höherem Hb-Gehalt die Photometergrenze erreicht wird. Dies kann zwar ausreichend für die Beseitigung von Hämolysestörungen sein, ist jedoch keinesfalls ausreichend für die Beseitigung von Blutersatzmittel-Störungen.

Eine weitere Möglichkeit zur Beseitigung von Hämoglobinstörungen wurde für die Bestimmung von Albumin publiziert (PCT-Anmeldung WO 97/45728), wo durch spezielle Kombinationen von Haupt- und Nebenwellenlängen eine Beseitigung von Hämoglobinstörungen erzielt werden konnte. Die hier genannten Wellenlängenkombinationen können jedoch nicht für die Bestimmung der alkalischen Phosphatase verwendet werden, da bei diesen Wellenlängen kein Meßsignal mehr für 4-Nitrophenol erhalten wird.

Die Offenlegungsschrift WO 97/45733 beschreibt, daß mit den Nebenwellenlängen 546 und 570 nm bei einzelnen UV-Tests die Störungen durch Hämoglobin beseitigt werden können. Dieses Verfahren ist jedoch nur auf enzymatische UV-Tests mit einer Hauptmeßwellenlänge von 340 nm anwendbar. Während hierbei eine vollständige Hb-Entstörung allein durch die Nebenwellenlängen 546 bzw. 570 nm erreicht werden kann, ist dies im Fall von enzymatischen Farbtests wie beispielsweise zur Bestimmung von alkalischer Phosphatase, bei denen die Hauptmeßwellenlänge im Bereich von 415 nm liegt, nicht möglich.

Im US-Patent 5,766,872 wird erwähnt, daß bei der Amylase-Bestimmung die Nebenwellenlänge von 577 nm zu einer Verringerung der Hämolysestörung führt. Aus den angeführten Meßdaten ist jedoch zu erkennen, daß bereits bei einem Hb-Gehalt von 500 mg/dl eine signifikante Meßwertabweichung von bis zu 8 % vorliegt. Das reicht zwar für die Härnolyse-Entstörung, doch ist aufgrund der verwendeten Hauptmeßwellenlänge von ca. 415 nm zu erwarten, daß bei höherem Hb-Gehalt (wie er bei einer Therapie mit Blutersatzmitteln auftritt) diese Meßwertabweichung ebenfalls größer wird und eine ausreichende Hb-Entstörung dann nicht mehr gegeben ist.

In der DE-A-196 28 484 wird ein Verfahren zur Beseitigung von Störungen, die durch die Anwesenheit von freiem Hämoglobin hervorgerufen werden, beschrieben. Die Hämoglobin-Störung bei Photometrischen Messungen wie beispielsweise der Bestimmung von alkalischer Phosphatase wird dadurch beseitigt, dass der zu vermessenen Probe peroxidische Verbindungen zugesetzt werden. Dadurch kommt es zu einer raschen Ausbleichung der vom Hämoglobin verursachten Färbung und Wegfall der damit verbundenen spektralen Störungen. Die Messung der mit Peroxid behandelten Probe erfolgt bei Wellenlängen von etwa 380-450nm und/oder 520-590nm. Die Bestimmung von alkalischer Phosphatase ohne Peroxid-Behandlung wird weder offenbart noch nahegelegt.

Im Stand der Technik ist kein Verfahren zur Bestimmung von alkalischer Phosphatase ohne Peroxid-Zugabe bekannt, das auch in Anwesenheit von hohen Hb-Konzentrationen, wie sie beispielsweise in Blutersatzmittel enthaltenden Proben vorkommen, störungsfrei durchgeführt werden kann.

Aufgabe war es daher, ein verbessertes Verfahren zur Bestimmung der alkalischen Phosphatase in einer Probe zu entwickeln, das die Nachteile des Standes der Technik weitgehend überwindet. Insbesondere sollte ein einfaches und anwenderfreundliches Verfahren zur Beseitigung von Störungen durch Hämoglobin und auf Hämoglobin basierenden Blutersatzmitteln bei der Bestimmung der alkalischen Phosphatase bereitgestellt werden.

Gelöst wird die Aufgabe durch das in den Ansprüchen näher definierte Verfahren zur Bestimmung von alkalischer Phosphatase in einer Probe durch optische Messung. Das Verfahren ist dadurch gekennzeichnet, daß man eine Hauptmeßwellenlänge von 450 ± 10 nm und als Nebenmeßwellenlänge mindestens eine der Wellenlängen 480 ± 10 nm, 546 ± 10 nm oder 575 ± 10 nm verwendet. Bevorzugt wird nur eine der genannten Nebenmeßwellenlängen verwendet.

Es hat sich überraschenderweise gezeigt, daß eine effektive Hb-Entstörung für die Bestimmung der alkalischen Phosphatase dann möglich ist, wenn zum einen die Hauptwellenlänge und zusätzlich dazu auch die Nebenwellenlänge geändert wird. Für eine zufriedenstellende Hb-Entstörung ist es nicht ausreichend, nur die Hauptwellenlänge oder nur die Nebenwellenlänge zu ändern.

Aufgrund des Absorptionsspektrums von 4-Nitrophenol kann die alkalische Phosphatase nicht nur bei 415 nm gemessen werden, sondern auch bei 450 ± 10 nm. Zwar befindet sich damit die Hauptmeßwellenlänge nicht im üblicherweise verwendeten Absorptionsmaximum der Nachweisreaktion, sondern an dessen Flanke, aber das erhaltene Meßsignal ist dennoch ausreichend für die exakte Bestimmung der alkalischen Phosphatase.

Bereits durch Wahl der neuen Hauptmeßwellenlänge 450 ± 10 nm kommt es zu einer leichten Verringerung der Hämoglobin-Störung, aber eine vollständige Entstörung erhält man überraschenderweise erst durch die Kombination der Hauptwellenlänge 450 ± 10 nm mit mindestens einer der Nebenwellenlängen 480 ± 10 nm, 546 ± 10 nm oder 575 ± 10 nm. Als besonders geeignet hat sich eine Nebenwellenlänge von 570 nm erwiesen. Insbesondere für die Bestimmung der alkalischen Phosphatase nach der IFCC-Methode hat sich die Nebenwellenlänge 480 ± 10 nm zur Hb-Entstörung als gut geeignet erwiesen (Beispiel 2).

Andere Nebenwellenlängen wie beispielsweise 340, 376, 505, 600, 660 und 700 nm haben sich zur Hämoglobin-Entstörung als nicht geeignet erwiesen.

Mit Hilfe des erfindungsgemäßen Verfahrens ist es erstmals auf einfache Weise möglich, die durch Hämoglobin bzw. Hb-analoge Verbindungen verursachten Störungen bei der Bestimmung von alkalischer Phosphatase bis zu einem Hb-Gehalt von mind. 3000 mg/dl zu beseitigen. Die Obergrenze zur Beseitigung der Hb-Störungen liegt bei der durch die Leistungsfähigkeit des verwendeten Photometers bestimmten Grenze. Bis zu 6500 mg/dl Hämoglobin-Gehalt ist daher eine gute Entstörung durch das erfindungsgemäße Verfahren denkbar. Die Erfindung ist weiterhin für die verschiedenen Reagenzien zur Bestimmung von alkalischer Phosphatase anwendbar, wie aus den Beispielen 1 bis 3 ersichtlich ist.

Das erfindungsgemäße Verfahren eignet sich zur Bestimmung beliebiger Proben, in denen freies Hämoglobin vorliegt. Der Begriff "freies Hämoglobin" im Sinne der Erfindung wird in Abgrenzung zu solchem Hämoglobin verwendet, das in intakten Erythrocyten enthalten ist. Beispiele für Proben, die freies Hämoglobin enthalten, sind hämolytische Serum- oder Plasmaproben oder Proben, die Blutersatzmittel enthalten. Beispiele für Blutersatzmittel, die im Sinne der vorliegenden Erfindung unter den Begriff "freies Hämoglobin" fallen, sind derivatisierte, polymerisierte, modifizierte oder quervernetzte Derivate von Hämoglobinen, insbesondere von Humanhämoglobin oder Rinderhämoglobin. z. B. DCL-Hämoglobin (Diaspirin-crosslinked Hämoglobin), sowie rekombinant hergestelltes Hämoglobin.

Ebenfalls ein Gegenstand der Erfindung ist eine Methode zur Beseitigung von Störungen, die durch freies Hämoglobin hervorgerufen werden, in einem Verfahren zur Bestimmung von alkalischer Phosphatase. Die Methode ist dadurch gekennzeichnet, daß man eine Hauptmeßwellenlänge von 450 ± 10 nm und als Nebenmeßwellenlänge mindestens eine der Wellenlängen 480 ± 10 nm, 546 ± 10 nm oder 575 ± 10 nm verwendet.

Ein weiterer Gegenstand der Erfindung ist die Verwendung einer Hauptmeßwellenlänge von 450 ± 10 nm in Kombination mit mindestens einer der Nebenmeßwellenlängen 480 ± 10 nm, 546 ± 10 nm oder 575 ± 10 nm zur Beseitigung von Störungen durch freies Hämoglobin oder durch auf Hämoglobinbasis hergestellte Blutersatzmittel in einem Verfahren zur Bestimmung von alkalischer Phosphatase.

Die Erfindung wird durch nachfolgende Beispiele erläutert:

### Beispiele

### Allgemeine Methoden:

Ein Teil eines Serumpools wurde mit einer Hb-haltigen Lösung derart versetzt, daß ein Hb-Gehalt von mindestens 3000 mg/dl erreicht wurde. Ein anderer gleichgroßer Teil desselben Serumpools wurde mit der äquivalenten Menge NaCl-Lösung (154 mmol/l) versetzt. Beide Teile wurden anschließend in unterschiedlichem Verhältnis derart miteinander vermischt, daß eine Hb-Konzentrationsreihe aus 11 Proben erhalten wird, deren niedrigste Probe kein Hb und deren höchste Probe mindestens 3000 mg/dl Hb enthält.

### Beispiel 1

### Bestimmung der Alkalischen Phosphatase nach der SFBC-Methode

Bestimmung nach Empfehlungen der Société Française de Biologie Clinique gemäß Ann. Biol. Clin. Vol 35, 271 (1977)

Die Bestimmung wurde an einem Boehringer Mannheim/Hitachi 911-Analysengerät durchgeführt.

Es wurden folgende Reagenzien verwendet:

| | |
|---|---|
| Reagenz 1: | 930 mmol/l 2-Amino-2-methy-1-propanol-Puffer, pH 10.5; |
| | 1.03 mmol/l Magnesiumaspartat |
| Reagenz 2: | 930 mmol/l 2-Amino-2-methyl-1-propanol-Puffer, pH 10.5; |
| | 1.03 mmol/l Magnesiumaspartat; 98 mmol/l 4-Nitrophenylphosphat |

Die Testdurchführung war wie folgt: Zu 11 µl Probe wurden 250 µl Reagenz 1 und nach 5 min 50 µl Reagenz 2 gegeben. Die Bestimmung des Analyten erfolgte nach einer Dauer von weiteren 50 sec über einen Zeitraum von 4 min, wobei die Extinktionsänderung während dieser 4 min gemessen wurde. Zur Messung wurden Kombinationen folgender Hauptmeßwellenlängen (λ₁) und Nebenmeßwellenlängen (λ₂) verwendet: λ₁/λ₂ = 415/546 nm, 415/570 nm, 415/660 nm und 450/660 nm (Vergleich) sowie 450/546 nm und 450/570 nm (Erfindung).

Als weiterer Vergleich wurde die Bestimmung der Alkalischen Phosphatase nach der von Jay und Provasek erwähnten Rate-Blank-Messung durchgeführt (als "415/660 nm RB" bezeichnet).

Das Ergebnis ist in Tabelle 1 gezeigt. Es ist zu erkennen, daß bei Verwendung der erfindungsgemäßen Meßwellenlängenkombinationen von 450/546 nm bzw. 450/570 nm der Hämoglobin-Einfluß im Vergleich zu den anderen Meßwellenlängenkombinationen bzw. zur Rate-Blank-Messung signifikant reduziert wird.

**Tabelle 1**

| Gemessener Gehalt an Alkalischer Phosphatase bei 37°C in U/l | | | | | | | |
|---|---|---|---|---|---|---|---|
| Hb- | 415/546 | 415/570 | 415/660 | 415/660 | 450/660 | 450/546 | 450/570 |
| Gehalt* | nm | nm | nm | nm RB | mm | nm | nm |
| [mg/dl] | | | | | | | |
| 0 | 42 | 42 | 42 | 42 | 42 | 41 | 42 |
| 300 | 33 | 32 | 32 | 44 | 35 | 40 | 41 |
| 600 | 24 | 24 | 22 | 46 | 29 | 40 | 40 |
| 900 | 16 | 16 | 14 | 46 | 24 | 39 | 41 |
| 1200 | 11 | 11 | 8 | 44 | 22 | 40 | 40 |
| 1500 | 7 | 7 | 3 | 5 | 19 | 40 | 40 |
| 1800 | 2 | 2 | -2 | 0 | 17 | 39 | 40 |
| 2100 | 3 | 3 | -2 | 0 | 17 | 40 | 41 |
| 2400 | 3 | 3 | -2 | 1 | 15 | 43 | 42 |
| 2700 | 4 | 3 | -1 | 0 | 16 | 42 | 43 |
| 3000 | 4 | 3 | -2 | 1 | 19 | 45 | 45 |

| | | | | | | | |
|---|---|---|---|---|---|---|---|
| * hier wurde ein vernetztes Hämoglobin eingesetzt | | | | | | | |

### Beispiel 2

### Bestimmung der Alkalischen Phosphatase nach der IFCC-Methode

Bestimmung nach Empfehlungen der International Federation of Clinical Chemistry gemäß J. Clin. Chem. Clin. Biochem. Vol 21, 731-748 (1983)

Die Bestimmung wurde an einem Boehringer Mannheim/Hitachi 911-Analysengerät durchgeführt.

Es wurden folgende Reagenzien verwendet:

| | |
|---|---|
| Reagenz 1: | 360 mmol/l 2-Amino-2-methyl-1-propanol-Puffer, pH 10,4 (30°C); |
| | 2,04 mmol/l Magnesiumacetat; 1,02 mmol/l Zinksulfat; |
| | 2,04 mmol/l N-(2-Hydroxyethyl)-ethylendiamintriessigsäure) |
| Reagenz 2: | 360 mmol/l 2-Amino-2-methyl-1-propanol-Puffer, pH 10,4 (30°C); |
| | 2,04 mmol/l Magnesiumacetat; 1,02 mmol/l Zinksulfat; |
| | 2,04 mmol/l N-(2-Hydroxyethyl)-ethylendiamintriessigsäure) |
| | 104 mmol/l 4-Nitrophenylphosphat |

Die Testdurchführung war wie folgt: Zu 7 µl Probe wurden 250 µl Reagenz 1 und nach 5 min 60 µl Reagenz 2 gegeben. Die Bestimmung des Analyten erfolgte nach einer Dauer von weiteren 50 sec über einen Zeitraum von 4 min, wobei die Extinktionsänderung während dieser 4 min gemessen wurde. Zur Messung wurden Kombinationen folgender Hauptmeßwellenlängen (λ₁) und Nebenmeßwellenlängen (λ₂) verwendet: λ₁/λ₂ = 415/700 nm (Vergleich) sowie 450/480 nm (Erfindung).

Das Ergebnis ist in Tabelle 2 gezeigt. Es ist zu erkennen, daß bei Verwendung der erfindungsgemäßen Meßwellenlängenkombination von 450/480 nm der Hämoglobin-Einfluß im Vergleich zur bisherigen Meßwellenlängenkombination 415/700 nm signifikant reduziert wird und auch bei sehr hohem Hb-Gehalt keine negativen Werte auftreten.

**Tabelle 2**

| Gemessener Gehalt an Alkalischer Phosphatase bei 37°C in U/l | | |
|---|---|---|
| Hb-Gehalt* | 415/700 nm | 450/480 nm |
| [mg/dl] | | |
| 0 | 167 | 170 |
| 300 | 157 | 164 |
| 600 | 152 | 160 |
| 900 | 145 | 157 |
| 1200 | 139 | 154 |
| 1500 | 133 | 151 |
| 1800 | 126 | 149 |
| 2100 | 123 | 150 |
| 2400 | 116 | 153 |
| 2700 | -4,3 | 154 |
| 3000 | -4,4 | 153 |

| | | |
|---|---|---|
| * hier wurde ein rekombinant hergestelltes Hämoglobin eingesetzt | | |

### Beispiel 3

### Bestimmung der Alkalischen Phosphatase nach der DGKC-Methode

Bestimmung nach Empfehlungen der Deutschen Gesellschaft für Klinische Chemie gemäß Z. Klin. Chem. Klin. Biochem. Vol 10, 290 (1972)

Die Bestimmung wurde an einem Boehringer Mannheim/Hitachi 911-Analysengerät durchgeführt.

Es wurden folgende Reagenzien verwendet:

| | |
|---|---|
| Reagenz 1: | 1.02 mmol/l Diäthanolamin-Puffer, pH 9.8; 0,51 mmol/l Magnesiumchlorid |
| Reagenz 2: | 1,02 mmol/l Diäthanolamin-Puffer, pH 9,8; 0,51 mmol/l Magnesiumchlorid; |
| | 61 mmol/l 4-Nitrophenylphosphat |

Die Testdurchführung war wie folgt: Zu 4 µl Probe wurden 250 µl Reagenz 1 und nach 5 min 50 µl Reagenz 2 gegeben. Die Bestimmung des Analyten erfolgte nach einer Dauer von weiteren 50 sec über einen Zeitraum von 4 min, wobei die Extinktionsänderung während dieser 4 min gemessen wurde. Zur Messung wurden Kombinationen folgender Hauptmeßwellenlängen (λ₁) und Nebenmeßwellenlängen (λ₂) verwendet: λ₁/λ₂ = 415/700 nm (Vergleich) sowie 450/546 nm (Erfindung).

Das Ergebnis ist in Tabelle 3 gezeigt. Es ist zu erkennen, daß bei Verwendung der erfindungsgemäßen Meßwellenlängenkombination von 450/546 nm der Hämoglobin-Einfluß im Vergleich zur bisherigen Meßwellenlängenkombination 415/700 nm signifikant reduziert wird und auch bei sehr hohem Hb-Gehalt keine negativen Werte auftreten.

**Tabelle 3**

| Gemessener Gehalt an Alkalischer Phosphatase bei 37°C in U/l | | |
|---|---|---|
| Hb-Gehalt* [mg/dl] | 415/700 nm | 450/546 nm |
| 0 | 287 | 298 |
| 650 | 226 | 279 |
| 1300 | 172 | 277 |
| 1950 | 119 | 278 |
| 2600 | 65 | 278 |
| 3250 | 20 | 278 |
| 3900 | -25 | 280 |
| 4550 | -65 | 284 |
| 5200 | -100 | 286 |
| 5850 | -13 | 286 |
| 6500 | -15 | 302 |

| | | |
|---|---|---|
| * hier wurde ein polymerisiertes Hämoglobin eingesetzt | | |

## Patentansprüche

1. Verfahren zur Bestimmung von alkalischer Phosphatase in einer Probe durch optische Messung von p-Nitrophenol **dadurch gekennzeichnet, daß** man eine Hauptmeßwellenlänge von 450 ± 10 nm und als Nebenmeßwellenlänge mindestens eine der Wellenlängen 480 ± 10 nm, 546 ± 10 nm oder 575 ± 10 nm verwendet.

2. Verfahren nach Anspruch 1, **dadurch gekennzeichnet, daß** man als Nebenmeßwellenlänge 480 ± 10 nm verwendet.

3. Verfahren nach Anspruch 1, **dadurch gekennzeichnet, daß** man als Nebenmeßwellenlänge 546 ± 10 nm verwendet.

4. Verfahren nach Anspruch 1, **dadurch gekennzeichnet, daß** man als Nebenmeßwellenlänge 575 ± 10 nm verwendet.

5. Verfahren nach Anspruch 1, **dadurch gekennzeichnet, daß** man als Nebenmeßwellenlänge 570 nm verwendet

6. Verfahren nach einem der vorhergehenden Ansprüche, **dadurch gekennzeichnet, daß** die Bestimmung in einer Serum- oder Plasmaprobe durchgeführt wird.

7. Verfahren nach einem der vorhergehenden Ansprüche, **dadurch gekennzeichnet, daß** man eine Probe bestimmt, die freies Hämoglobin oder ein auf Hämoglobinbasis hergestelltes Blutersatzmittel enthält.

8. Verfahren nach Anspruch 7, **dadurch gekennzeichnet, daß** das Blutersatzmittel ein derivatisiertes, polymerisiertes, modifiziertes oder quervernetztes Humanhämoglobin, Rinderhämoglobin oder ein rekombinant hergestelltes Hämoglobin enthält.

9. Verfahren nach einem der vorhergehenden Ansprüche, **dadurch gekennzeichnet, daß** die Probe einen Hämoglobingehalt bis zu 6500 mg/dl aufweist.

10. Methode zur Beseitigung von Störungen, die durch freies Hämoglobin oder Blutersatzmittel hervorgerufen werden, in einem Verfahren zur Bestimmung von alkalischer Phosphatase gemäß Anspruch 1 **dadurch gekennzeichnet, daß** man eine Hauptmeßwellenlänge von 450 ± 10 nm und als Nebenmeßwellenlänge mindestens eine der Wellenlängen 480 ± 10 nm, 546 ± 10 nm oder 575 ± 10 nm verwendet.

11. Verwendung einer Hauptmeßwellenlänge von 450 ± 10 nm in Kombination mit mindestens einer der Nebenmeßwellenlängen 480 ± 10 nm, 546 ± 10 nm oder 575 ± 10 nm zur Beseitigung von Störungen durch freies Hämoglobin oder Blutersatzmittel in einem Verfahren gemäß Anspruch 1 zur Bestimmung von alkalischer Phosphatase.

## Claims

1. Procedure for the determination of alkaline phosphatase in a sample by optical measurement wherein a main measuring wave length of 450 ± 10 nm and as a secondary measuring wave length at least one of the wave lengths 480 ± 10 nm, 546 ± 10 nm or 575 ± 10 nm are used.

2. Procedure as claimed in claim 1 wherein a secondary measuring wave length of 480 ± 10 nm is used.

3. Procedure as claimed in claim 1 wherein a secondary measuring wave length of 546 ± 10 nm is used.

4. Procedure as claimed in claim 1 wherein a secondary measuring wave length of 575 ± 10 nm is used.

5. Procedure as claimed in claim 1 wherein a secondary measuring wave length of 570 nm is used.

6. Procedure as claimed in one of the aforementioned claims wherein the determination is performed in a serum or plasma sample.

7. Procedure as claimed in one of the aforementioned claims wherein a sample containing free hemoglobin or a blood substitute produced on the basis of hemoglobin is analyzed.

8. Procedure as claimed in claim 7 wherein the blood substitute contains a derivatized, polymerized, modified or cross-linked human hemoglobin, bovine hemoglobin or a recombinantly produced hemoglobin.

9. Procedure as claimed in one of the aforementioned claims wherein the sample has a hemoglobin concentration of up to 6500 mg/dl.

10. Method for the elimination of interferences caused by free hemoglobin or blood substitutes, in a procedure for the determination of alkaline phosphatase wherein a main measuring wave length of 450 ± 10 nm and as a secondary measuring wave length at least one of the wave lengths 480 ± 10 nm, 546 ± 10 nm or 575 ± 10 nm is used.

11. Use of a main measuring wave length of 450 ± 10 nm combined with at least one of the secondary measuring wave lengths 480 ± 10 nm, 546 ± 10 nm or 575 ± 10 nm for elimination of interferences caused by free hemoglobin or blood substitutes in a procedure for the determination of alkaline phosphatase.

## Revendications

1. Procédé pour la détermination de la teneur en phosphatase alcaline dans un échantillon par mesure optique de p-nitrophénol, **caractérisé en ce qu'**on utilise une longueur d'onde de mesure principale de 450 ± 10 nm et, à titre de longueur d'onde de mesure secondaire, au moins une des longueurs d'onde de 480 ± 10 nm, 546 ± 10 nm ou 575 ± 10 nm.

2. Procédé selon la revendication 1, **caractérisé en ce qu'**on utilise, à titre de longueur d'onde de mesure secondaire, une longueur d'onde de 480 ± 10 nm.

3. Procédé selon la revendication 1, **caractérisé en ce qu'**on utilise, à titre de longueur d'onde de mesure secondaire, une longueur d'onde de 546 ± 10 nm.

4. Procédé selon la revendication 1, **caractérisé en ce qu'**on utilise, à titre de longueur d'onde de mesure secondaire, une longueur d'onde de 575 ± 10 nm.

5. Procédé selon la revendication 1, **caractérisé en ce qu'**on utilise, à titre de longueur d'onde de mesure secondaire, une longueur d'onde de 570 nm.

6. Procédé selon l'une quelconque des revendications précédentes, **caractérisé en ce qu'**on procède à la détermination dans un échantillon de sérum ou de plasma.

7. Procédé selon l'une quelconque des revendications précédentes, **caractérisé en ce qu'**on détermine un échantillon qui contient de l'hémoglobine libre ou bien un succédané du sang préparé à base d'hémoglobine.

8. Procédé selon la revendication 7, **caractérisé en ce que** le succédané du sang contient une hémoglobine humaine, une hémoglobine bovine ou une hémoglobine préparée par voie recombinante, dérivée, polymérisée, modifiée ou réticulée.

9. Procédé selon l'une quelconque des revendications précédentes, **caractérisé en ce que** l'échantillon présente une teneur en hémoglobine s'élevant jusqu'à 6500 mg/dl.

10. Procédé pour supprimer des parasites qui sont dus à la présence d'hémoglobine libre ou d'un succédané du sang, dans un procédé pour la détermination de la teneur en phosphatase alcaline selon la revendication 1, **caractérisé en ce qu'**on utilise une longueur d'onde de mesure principale de 450 ± 10 nm et, à titre de longueur d'onde de mesure secondaire, au moins une des longueurs d'onde de 480 ± 10 nm, 546 ± 10 nm ou 575 ± 10 nm.

11. Utilisation d'une longueur d'onde de mesure principale de 450 ± 10 nm en combinaison avec au moins une des longueurs d'ondes de mesure secondaires de 480 ± 10 nm, 546 ± 10 nm ou 575 ± 10 nm pour supprimer des parasites qui sont dus à la présence d'hémoglobine libre ou d'un succédané du sang, dans un procédé selon la revendication 1 pour la détermination de la teneur en phosphatase alcaline.
